# EUROPEAN PATENT SPECIFICATION

(11) **EP 0 438 078 B1**
(45) Date of publication and mention of the grant of the patent: **05.06.1996**
(21) Application number: 91100236.8
(22) Date of filing: 09.01.1991
(51) Int. Cl.: A61N 1/30, A61N 25/00

(54) **Iontophoresis device**
Iontophoresegerät
Appareil d'iontophorèse

(30) Priority: 15.01.1990 IT 4000790; 27.07.1990 IT 2110790
(43) Date of publication of application: 24.07.1991
(73) Proprietor: Rossi, Cino, I-00123 Roma (IT); Eruzzi, Silvio, I-46100 Mantova (IT)
(72) Inventor: Rossi, Cino, I-00123 Roma (IT); Eruzzi, Silvio, I-46100 Mantova (IT)
(74) Representative: Modiano, Guido, Dr.-Ing.

(56) References cited:
- DE-A- 3 809 815
- FR-A- 2 516 388
- US-A- 3 324 847

## Description

The present invention relates to a device for carrying out iontophoresis, in particular intracorporeal iontophoresis.

Iontophoresis is a per se known method for the administration of drugs by means of the transfer of ions through the intact skin under the effect of the application of electric current to a liquid medium containing said ions.

Iontophoresis has so far been used mostly for extracorporeal applications.

Generic proposals have also been made for the use of iontophoresis in intracorporeal applications by introducing, through natural pathways, adequate and appropriately insulated electrodes and drug solutions in intracorporeal cavities such as the bladder, the prostatic duct, the colon etc. and by subsequently applying an electric current. Thus, for example, THIEL describes in DOS 3844518 the application of iontophoresis to the treatment of the bladder by means of the use of a tubular electrode which is insulated at the tip and through which the solution of the drug is supplied; said electrode apparently has two openings for connection between the inside of the electrode and its outside. With such an electrode, there is the risk during its use that it may make contact with the pulsating movable wall of the organ being treated, for example the bladder, causing electric burns thereto.

From DE-A-3 809 815 it is known a device for carrying out iontophoresis as defined in the preamble of claim 1.

From US-A-3 324 847 it is known catheter for therapeutic use having an inflatable bag suitable to avoid, when inflated, the removal of the catheter from a cavity of a patient's body.

Due to these problems and due to a reduced effectiveness of the performed iontophoresis, these generic proposals have not produced devices of practical applicability.

The aim of the present invention is to provide a device for carrying out iontophoresis which obviates the disadvantages which can be observed in the devices proposed so far, most of all in application in intracorporeal iontophoresis.

In particular, a specific object of the present invention is to provide an iontophoresis device which has no risk of damaging the tissues as a consequence for example of accidental contacts of its conducting parts with tissues.

A further object is to provide an iontophoresis device which ensures high effectiveness of the iontophoresis treatment, achieving a complete transfer of the drug ions to the organs being treated in the shortest possible time.

Still another object of the invention is to provide an iontophoresis device which also ensures a complete contact between the electrode and the medicated solution, so as to fully exploit the entire active surface of the electrode and prevent its local wear and consequent undesirable secondary reactions caused by the exposure of substrate materials or even a breakage of the worn electrode.

Therefore, ultimately, a further object of the invention is also to provide an iontophoresis device which is effective and safe in operation, with a minimum use of active material for the electrode.

This aim, these objects and others which will become apparent from the following description are achieved by a device for performing intracorporeal iontophoresis as defined in claim 1.

Further characteristics and advantages of the invention will become apparent from the following description, given by way of non-limitative example with reference to the accompanying drawings, wherein:
figures 1 to 4 are views of a iontophoresis device
figure 5 is a view of a further embodiment of the iontophoresis device according to the invention in disassembled condition;
figure 6 is an enlarged axial sectional cutout view of the catheter of the device of figure 5;
figure 7 is a view of a further embodiment of the device according to the invention; and
figures 8 and 9 are axial sectional cutout views of two further embodiments of the device according to the invention.

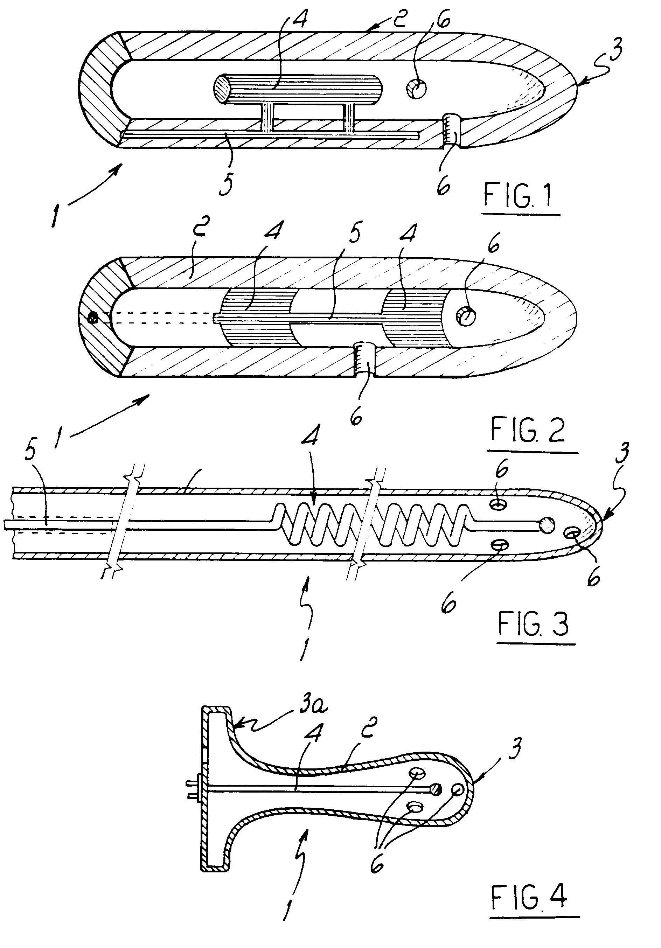

With reference to figures 1 to 4, the iontophoresis device comprises a tubular catheter 1, made of electrically insulating and flexible material, which has a terminal portion 2 provided for intracorporeal insertion in use. It is possible, for example, to use a conventional catheter for medical uses, generally made of flexible and mechanically resistant plastic material. The distal end 3 of the terminal portion 2 is closed, so as to make the internal cavity of the tubular catheter suitable for containing liquids, in particular an iontophoresis medium, such as a solution of a drug in ionized form. The liquid iontophoresis medium is fed into the catheter from its extracorporeal end (not illustrated) or from any opening provided on the extracorporeal portion of the catheter.

The terminal portion 2 of the catheter 1 is shaped so that it is anatomically compatible with the placement point: figures 1, 2 and 3 illustrate an elongated and narrow shape for use for example in the bladder/prostatic duct or in otorhinolaryngological use, or furthermore in peritoneal or abdominal use, whereas in figure 4 the end 3 is slightly enlarged and the opposite end defines a bottom 3a for gynecological or rectal use.

Connecting openings or holes 6 are defined on the terminal portion 2 of the catheter and allow the flow of the liquid iontophoresis medium and the migration of the drug ions from the internal cavity of the catheter into the body cavity or organ which is subjected to the treatment.

Inside the catheter there is an electrode 4 which can be variously configured and is appropriately connected to an external current generator (not illustrated) and to a counterelectrode which is appropriately placed on the outside of the body, on the skin, as close as possible to the organ subjected to iontophoretic treatment.

Figures 1 to 4 illustrate various possible forms of shaped electrodes, for example respectively stub-like, plate-like, spiral-like or wire-like ones. The connection of the electrodes to the electric circuit which is external to the catheter or between separate parts thereof is provided by means of appropriate conductors 5 which pass through the internal cavity of the catheter or are embedded in its wall, as illustrated in figure 1.

According to the particular case, and in particular according to the specific iontophoretic treatment to be performed and to the particular condition of the patient, the electrode may be made of various materials. For example, it is possible to use cathodes made of materials which react with ions which are present in the iontophoresis medium or in the biological medium in which the iontophoresis is possibly performed. An example of these is silver, which is preferred when there are Cl⁻ ions in the iontophoresis medium, for example as counterions of the active drug ion. According to well-known electrophoresis and electrolysis mechanisms, when the iontophoresis current flows, the silver of the anode reacts with the chloride according to the equation 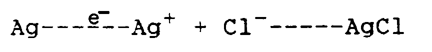
and the silver chloride thus formed deposits on the anode, forming in practice an anode made of Ag-AgCl.

In certain iontophoretic treatments it may be advantageous to use an anode which undergoes dissolution under the effect of the electric current, releasing into the iontophoresis medium its own ions which are themselves suitable for acting as active drug. This can be for example the case of Zn or Cu electrodes, since said elements are useful in the treatment of certain fungal infections.

Finally, it is possible to use as electrodes inert materials which do not react nor get consumed during iontophoresis, such as for example Au, C, Pt, stainless steel, etc.

Preferably, the electrode for intracorporeal insertion is made of Au. The counterelectrode is preferably made of Au and/or graphite.

The electrode may be entirely constituted by the chosen electrically conductive material. However, since said materials are rather expensive, it is sometimes advantageous to use a substrate made of a less expensive material, for example stainless steel, and apply thereon a coating of the electrically conductive active material chosen for the specific treatment.

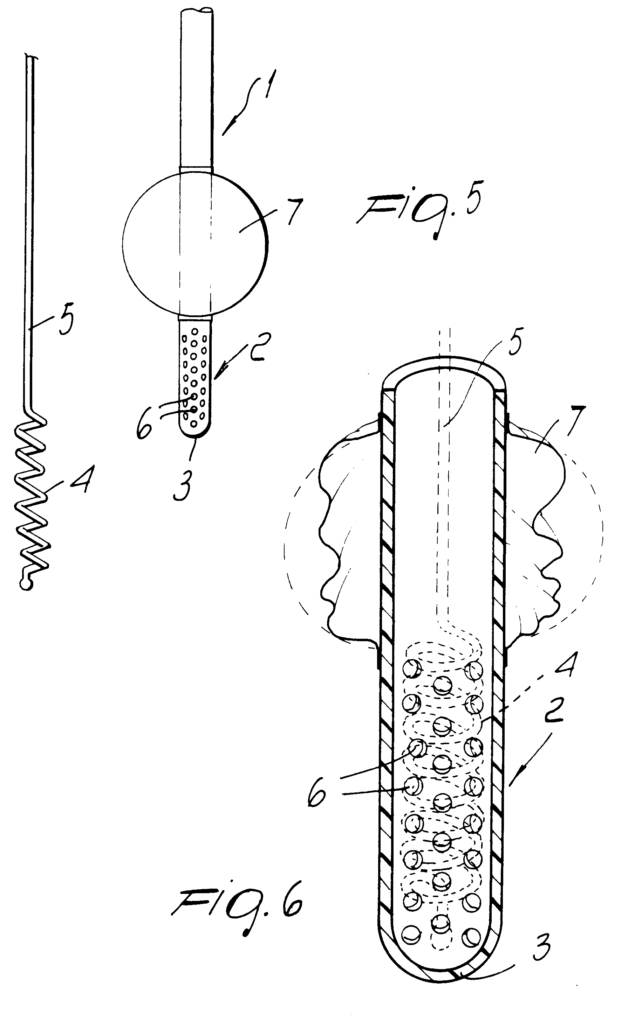

Figures 5 to 9 illustrate embodiments of the device according to the invention which are particularly advantageous, most of all in relation to the arrangement of the connecting holes 6 on the terminal portion 2 of the catheter 1.

Studies conducted by the Applicant have in fact shown that the number and arrangement of the holes 6 is critical for effectively carrying out iontophoresis. It has in fact been surprisingly observed that the surface of the electrode which is actively involved in the electrophoretic process is indeed determined by these factors. More specifically, in the case of electrodes which get consumed or react in the process, it has been visually noticed that with respect to the entire surface of the electrode, only that part thereof which extends at the portion of the catheter which is provided with the holes 6 is subjected to the phenomena of solubilization or forming of deposits such as those described above for Zn, Cu or Ag electrodes. Said localized phenomena are disadvantageous, since they can lead to unwanted side effects. For example, in the case of an electrode formed by an active coating on an inert substrate, the described phenomena localized on small surfaces of the electrode can lead to the erosion thereof until the substrate, which can trigger unwanted secondary reactions, is exposed. Also due to the only partial involvement of the electrode in the iontophoretic process, areas with an increased current density are created and can also give rise to unwanted secondary reactions, such as the hydrolysis of water in the presence of a copper or zinc electrode. Finally, in the case of electrodes which can get consumed in the process, localization of the iontophoretic phenomena can lead to the breakage of the electrodes themselves.

In order to obviate these undesirable phenomena and increase the effectiveness and efficiency of iontophoresis, it has been found to be particularly advantageous to arrange the holes 6 along a section of the terminal portion 2 which extends in correspondence with at least one part of the extension of the electrode in the internal cavity thereof. The holes 6 are preferably arranged along a section of the terminal portion which corresponds to a substantial part of the extension of the electrode, and even more preferably along a section of the catheter which corresponds to the entire extension of the electrode, as illustrated in the sectional view of figure 6.

It has furthermore been observed that it is extremely advantageous to arrange the holes 6 on the surface of the catheter with such a frequency that the distance between two successive and/or adjacent holes does not exceed twice the diameter of said holes or, in the case of non-circular holes, the average size of said holes. Holes with average diameters or sizes ranging from 7 to 3 mm are generally used.

Experimental results have shown that with an arrangement of the holes 6 as described above, the entire surface of the electrode is actively involved in the iontophoresis process, avoiding the undesirable localized phenomena described above. The iontophoresis treatment is furthermore achieved in a complete manner, with the transfer of the entire dose of drug used, in a considerably shortened time.

The catheter can be optionally furthermore provided with at least one bag 7, as illustrated in figures 5 and 6, which is fixed on its outer wall and can be reinflated in place in order to delimit and isolate the organ or part thereof subjected to treatment, so as to avoid the escape or dispersion of the active drug ions toward healthy parts.

The catheter can have a plurality of bags 7.

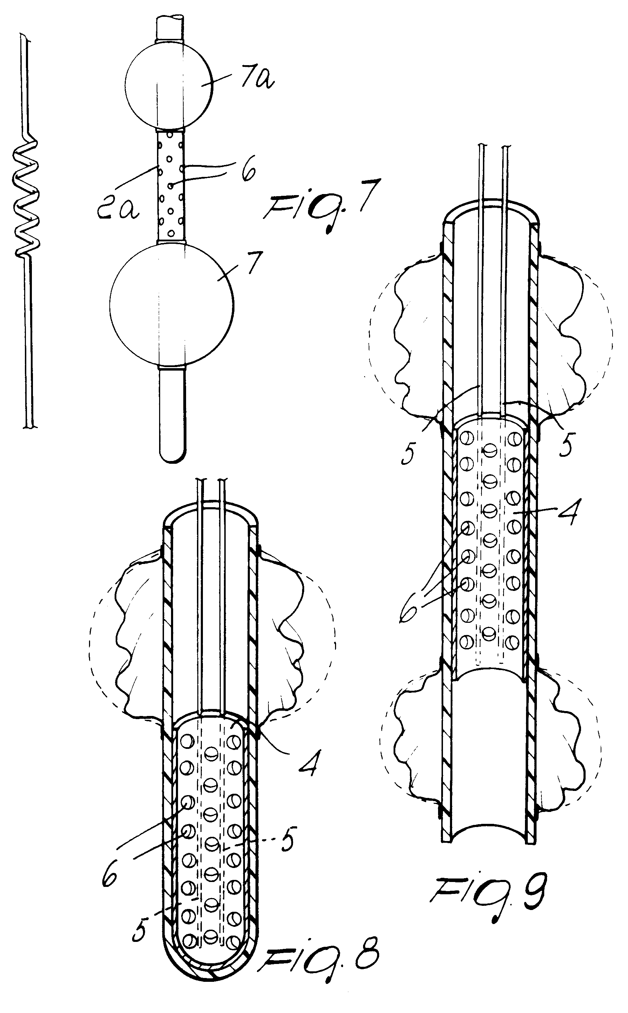

Thus, for example, figure 7 illustrates an iontophoresis device according to the invention provided with two reinflatable bags 7 and 7a which delimit the area to be treated inside the involved organ and also delimit an active section 2a on the terminal portion of the catheter; in use, the electrode extends at said section and the holes 6 are provided along said section with an arrangement according to the teachings of the present invention.

Figures 8 and 9 are views of two further embodiments of the device according to the invention, wherein the electrode 4 is constituted by a coating of electrically conductive material applied on the internal wall of the catheter which delimits the respective internal cavity and acts herein as support for the electrode. The electrode is connected to a current generator by means of conducting strips 5 which are embedded in the wall of the catheter in contact with the electrode 4 which is constituted by a coating. The active section of the device is delimited by a bag in figure 8, whereas it is delimited by two reinflatable bags in figure 9. The frequency and distribution of the holes 6 along the entire section of the catheter which is covered by the coating which acts as an electrode allows a uniform distribution of the iontophoresis medium in contact with the entire surface of the electrode, obtaining the already mentioned advantages.

As is evident from the above description, numerous other variations and modifications of the described iontophoresis devices are possible, and all are within the inventive concept described herein and claimed hereinafter.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Device for performing intracorporeal iontophoresis comprising a tubular catheter (1) having a terminal portion (2) which is closed at the respective end, suitable for being arranged intracorporeally during use, and provided with an internal cavity which can be supplied with a pharmacologically active liquid iontophoresis medium, and at least one electrode (4) which is located inside said terminal portion of the catheter and can be electrically connected to a current generator and to a counterelectrode, said terminal portion having a plurality of connecting holes (6) between the internal cavity of said catheter and its outside, characterized in that said connecting holes (6) are distributed along a section of said terminal portion (2) of said catheter (1) which corresponds at least to part of the extension of said electrode (4) in said internal cavity, said holes (6) being arranged on a section of said terminal portion (2) with such a frequency that the distance between two adjacent holes (6) does not exceed twice the average size of said holes.

2. Device according to claim 1, characterized in that said connecting holes (6) are distributed along a said section of the catheter (1) which corresponds to the entire extension of said electrode (4).

3. Device according to any one of claims 1 to 2, characterized in that said electrode (4) comprises an electrically conductive material which is inert with respect to the ions present in the iontophoresis medium.

4. Device according to claim 3, characterized in that said electrode (4) is made of gold.

5. Device according to claim 3, characterized in that said counterelectrode is made of gold and/or graphite.

6. Device according to any one of claims 1 to 2, characterized in that said electrode (4) comprises an electrically conductive material which is reactive in the iontophoresis process.

7. Device according to any one of the preceding claims, characterized in that said electrode (4) comprises a shaped electrically conductive element.

8. Device according to any one of claims 1 to 7, characterized in that said electrode (4) comprises a coating of electrically conductive material applied on a substrate.

9. Device according to claim 8, characterized in that said electrode (4) comprises an electrically conductive coating applied on the internal walls of the catheter (1) which delimit said internal cavity.

10. Device according to claim 1, characterized in that it comprises at least one reinflatable bag (7) which is fixed externally to said terminal portion (2) of the catheter (4) and is suitable for delimiting, in the inflated condition, at least one section of said catheter which is active in iontophoresis.

11. Device according to claim 10, characterized in that it comprises two of said reinflatable bags (7, 7a) which are arranged so as to be spaced and delimit, on said terminal portion (2), a section on which said connecting holes (6) are distributed.

## Patentansprüche

1. Vorrichtung zum Durchführen der körperinneren Iontophorese, mit einem rohrförmigen Katheter (1), dessen Endteil (2) an seinem Ende verschlossen ist, bei seiner Verwendung im Körperinneren angeordnet werden kann und mit einer inneren Kammer versehen ist, in die ein pharmakologisch aktives flüssiges Iontophoresemedium eingeführt werden kann, und mit mindestens einer Elektrode (4), die in dem Endteil des Katheters angeordndet ist und mit einem Stromgenerator sowie einer Gegenelektrode verbunden werden kann, wobei der Endteil mehrere Verbindungsöffnungen (6) zwischen der inneren Kammer des Katheters und der Außenseite hat, **dadurch gekennzeichnet**, daß die Verbindungsöffnungen (6) längs eines Abschnitts des Endteils (2) des Katheters (1) verteilt sind, der mindestens teilweise der Ausdehnung der Elektrode (4) in der inneren Kammer entspricht, und daß die Verbindungsöffnungen (6) auf dem Abschnitt des Endteils (2) so häufig angeordnet sind, daß der Abstand zwischen benachbarten Verbindungsöffnungen (6) ihre doppelte mittlere Größe nicht übersteigt.

2. Vorrichtung nach Anspruch 1, **dadurch gekennzeichnet**, daß die Verbindungsöffnungen (6) längs eines Abschnitts des Katheters (1) verteilt sind, der der gesamten Ausdehnung der Elektrode (4) entspricht.

3. Vorrichtung nach einem der Ansprüche 1 bis 2, **dadurch gekennzeichnet**, daß die Elektrode (4) aus einem elektrisch leitfähigen Material besteht, das bezüglich der in dem Iontophoresemedium vorhandenen Ionen neutral ist.

4. Vorrichtung nach Anspruch 3, **dadurch gekennzeichnet**, daß die Elektrode (4) aus Gold besteht.

5. Vorrichtung nach Anspruch 3, **dadurch gekennzeichnet**, daß die Gegenelektrode aus Gold und/oder Graphit besteht.

6. Vorrichtung nach einem der Ansprüche 1 bis 2, **dadurch gekennzeichnet**, daß die Elektrode (4) aus einem elektrisch leitfähigen Material besteht, das in dem Iontophoreseprozeß reaktionsfähig ist.

7. Vorrichtung nach einem der vorhergehenden Ansprüche, **dadurch gekennzeichnet**, daß die Elektrode (4) ein geformtes elektrisch leitfähiges Element hat.

8. Vorrichtung nach einem der Ansprüche 1 bis 7, **dadurch gekennzeichnet**, daß die Elektrode (4) eine Beschichtung aus elektrisch leitfähigem Material auf einem Substrat aufweist.

9. Vorrichtung nach Anspruch 8, **dadurch gekennzeichnet**, daß die Elektrode (4) eine elektrisch leitfähige Schicht an den die innere Kammer begrenzenden Innenwänden des Katheters (1) aufweist.

10. Vorrichtung nach Anspruch 1, **dadurch gekennzeichnet**, daß sie mindestens einen aufblasbaren, extern an dem Endteil (2) des Katheters (4) befestigten aufblasbaren Beutel (7) hat, der im aufgeblasenen Zustand mindestens einen bei der Iontophorese aktiven Abschnitt des Katheters begrenzt.

11. Vorrichtung nach Anspruch 10, **dadurch gekennzeichnet**, daß sie zwei aufblasbare Beutel (7, 7a) hat, die mit Abstand zueinander angeordnet sind und an dem Endteil (2) einen Abschnitt begrenzen, auf dem die Verbindungsöffnungen (6) verteilt sind.

## Revendications

1. Dispositif permettant d'effectuer une iontophorèse intracorporelle, comprenant un cathéter tubulaire (1) avec une partie terminale (2) qui est fermée au niveau de l'extrémité correspondante, apte à être disposé à l'intérieur du corps lors de l'utilisation et doté d'une cavité intérieure qui peut être alimentée en un milieu liquide d'iontophorèse, pharmacologiquement actif, et au moins une électrode (4) qui est située à l'intérieur de ladite partie terminale du cathéter et qui peut être reliée électriquement à un générateur de courant et à une contre-électrode, ladite partie terminale comportant une pluralité de trous de communication (6) entre la cavité intérieure dudit cathéter et l'extérieur de celui-ci,
caractérisé en ce que lesdits trous de communication (6) sont répartis le long d'un tronçon de ladite partie terminale (2) du cathéter (1) qui correspond à une partie au moins de la longueur de ladite électrode (4) dans ladite cavité intérieure, lesdits trous (6) étant disposés sur un tronçon de ladite partie terminale (2) avec une fréquence telle que la distance entre deux trous adjacents (6) n'excède pas le double de la taille moyenne desdits trous.

2. Dispositif selon la revendication 1, caractérisé en ce que lesdits trous de communication (6) sont répartis le long dudit tronçon de cathéter (1) qui correspond à toute la longueur de ladite électrode (4).

3. Dispositif selon l'une quelconque des revendications 1 à 2, caractérisé en ce que ladite électrode (4) comprend un matériau électro-conducteur qui est inerte par rapport aux ions présents dans le milieu d'iontophorèse.

4. Dispositif selon la revendication 3, caractérisé en ce que ladite électrode (4) est en or.

5. Dispositif selon la revendication 3, caractérisé en ce que ladite contre-électrode est en or et/ou en graphite.

6. Dispositif selon l'une quelconque des revendications 1 à 2, caractérisé en ce que ladite électrode (4) comprend un matériau électro-conducteur qui réagit dans le processus d'iontophorèse.

7. Dispositif selon l'une quelconque des précédentes revendications, caractérisé en ce que ladite électrode (4) comprend un élément électro-conducteur mis en forme.

8. Dispositif selon l'une des quelconque revendications 1 à 7, caractérisé en ce que ladite électrode (4) comprend un revêtement de matériau électro-conducteur appliqué sur un substrat.

9. Dispositif selon la revendication 8, caractérisé en ce que ladite électrode (4) comprend un revêtement électro-conducteur appliqué sur les parois intérieures du cathéter (1) qui délimitent ladite cavité intérieure.

10. Dispositif selon la revendication 1, caractérisé en ce qu'il comprend au moins une poche regonflable (7) qui est fixée à l'extérieur de ladite partie terminale (2) du cathéter (1) et qui permet de délimiter, lorsqu'elle est gonflée, au moins un tronçon dudit cathéter qui est actif dans l'iontophorèse.

11. Dispositif selon la revendication 10, caractérisé en ce qu'il comprend deux poches regonflables (7, 7a) qui sont disposées pour être espacées et délimiter, sur ladite partie terminale (2),un tronçon sur lequel lesdits trous de communication (6) sont répartis.
